# NEUE EUROPÄISCHE PATENTSCHRIFT

(11) **EP 0 904 788 B2**
(45) Veröffentlichungstag und Bekanntmachung des Hinweises auf die Entscheidung über den Einspruch: **08.11.2006**
(45) Hinweis auf die Patenterteilung: 12.11.2003
(21) Anmeldenummer: 98117768.6
(22) Anmeldetag: 18.09.1998
(51) Int. Cl.: A61M 1/16, G06F 9/44, G05B 15/02

(54) **Vorrichtung und Verfahren zur Bedienung medizintechnischer Geräte**
Apparatus and method of operating medico-technical devices
Dispositif et procédé de commande des appareils médicaux

(30) Priorität: 26.09.1997 DE 19742637
(43) Veröffentlichungstag der Anmeldung: 31.03.1999
(73) Patentinhaber: Fresenius Medical Care Deutschland GmbH, 61352 Bad Homburg v.d.H. (DE)
(72) Erfinder: Manke, Joachim, Dr., 35792 Löhnberg (DE); Schumacher, Gerhard, 35510 Butzbach (DE)
(74) Vertreter: Gossel, Hans K.

(56) Entgegenhaltungen:
- EP-A- 0 623 357
- US-A- 4 413 314
- US-A- 5 609 770
- US-A- 5 657 221
- Gebrauchsanweisung Dialysegerät "Dialog" der Firma B. Braun Melsungen AG Ausgabe 4/96
- Gebrauchsanweisung Dialysegerät AK 100 der Firma Gambro 6/96

## Beschreibung

Die vorliegende Erfindung betrifft eine Vorrichtung zur Bedienung medizintechnischer Geräte, insbesondere von Dialysegeräten, mit Bildschirm und Touchscreen-Oberfläche sowie mit Mitteln zur Anzeige und/oder Veränderung charakteristischer Behandlungsdaten. Die Erfindung betrifft ferner ein Verfahren zur Bedienung medizintechnischer Geräte.

Bildschirmeinheiten mitTouchscreen-Oberflächen zur Bedienung von medizintechnischen Geräten sind bekannt. Im allgemeinen werden derartige Bedieneinheiten verwendet, um die Istwerte von Patienten- oder Maschinenparametern durch Berühren eines entsprechenden Feldes der Touchscreen-Oberfläche abzufragen oder um entsprechende Sollwerte einzugeben.

Die EP 0 623 357 beschreibt eine Vorrichtung und ein Verfahren zur Dialyse, bei dem die Schnittstelle zwischen Gerät und Benutzer durch einen derartigen Touchscreen-Monitor realisiert ist. Der Monitor dient zur Abfrage und Eingabe charakteristischer Dialyse-Parameter, wie z.B. der Pumpgeschwindigkeit, der Ultrafiltrationsrate oder der Leitfähigkeit des Dialysates. Der Bediener muß zur Eingabe eines Sollwertes ein entsprechend mit der Bezeichnung des Parameters gekennzeichnetes Feld des Monitors berühren und gelangt schließlich in eine Bildschirmansicht, in der die Festlegung eines entsprechenden Wertes oder eines Profils möglich ist. Eine gattungsgemäße Vorrichtung und ein gattungsgemäßes Verfahren werden in der U. S. 5,609,770 offenbart. Hier erfolgt ebenfalls mit Hilfe eines Touchscreen-Monitors eine Auswahl der interessierenden Dialyse-Parameter. Nach der Auswahl kann den Parametern ein gewünschter Sollwert zugeordnet werden, wobei sowohl die Auswahl der Parameter als auch die Eingabe des Wertes über eine entsprechende Berührung der Oberfläche erfolgt. Aufgrund der Vielzahl der veränderbaren Dialyse-Parameter enthält eine Bildschirmansicht eine entsprechend große Anzahl von anwählbaren Feldern, was eine zuverlässige und rasche Bedienung des Gerätes erschwert. Ein fehlerfreies Arbeiten wird dadurch zusätzlich behindert, daß die Felder die gleiche Größe aufweisen und ausschließlich aufgrund ihrer Bezeichnung unterscheidbar sind. So ist es möglich, daß insbesondere unter Zeitdruck die falschen Felder der Touchscreen-Oberfläche berührt werden und somit beispielsweise eine fehlerhafte Eingabe eines Parameter-Sollwertes durchgeführt wird, was erhebliche Konsequenzen für die Gefährdung des Patienten während der Behandlung haben kann.

Es ist die Aufgabe der vorliegenden Erfindung die Handhabung von medizintechnischen Geräten zu vereinfachen und die Wahrscheinlichkeit für das Auftreten von Bedienungsfehlern zu verringern.

Diese Aufgabe wird ausgehend von einer gattungsgemäßen Vorrichtung und einem gattungsgemäßen Verfahren durch den kennzeichnenden Teil der Ansprüche 1 und 11 gelöst. Die erfindungsgemäße Vorrichtung zur Bedienung eines medizintechnischen Gerätes umfaßt einen Bildschirm und eine Touchscreen-Obertläche sowie erste Mittel zur Anzeige und/oder Veränderung charakteristischer Behandlungsdaten. Weiterhin sind zweite Mittel zur Darstellung von fürdie Komponenten des Gerätes charakteristischen Symbolen vorgesehen, wobei die ersten Mittel durch Berührung der Symbole auf der Touchscreen-Oberfläche ansteuerbar sind. Auf diese Weise wird erreicht, daß eine Verwechslung von Symbolen aufgrund mangelnder Unterscheidbarkeit der zu berührenden Felder der Touchscreen-Oberfläche weitgehend ausgeschlossen werden kann. Vielmehr wird durch einfaches Berühren des jeweiligen Symbols der zu beeinflussenden Komponente die Darstellung der entsprechenden Daten, beispielsweise in einem Kontrollfeld, aktiviert. Eine derartige graphische Benutzeroberfläche ermöglicht einen sicheren und zuverlässigen Gebrauch beispielsweise eines Dialysegerätes. Das medizinische Personal wird durch die charakteristischen Symbole sehr rasch mit dem Umfang der erfindungsgemäßen Vorrichtung vertraut, was zum einen die Einarbeitungszeit verringert und zum anderen die Zuverlässigkeit der Bedienung erhöht.

Besonders vorteilhaft ist es, wenn wenigstens ein vollständiger Dialysekreislauf mit den Symbolen der darin enthaltenen Komponenten darstellbar ist. Dabei können entweder der Kreislauf der Dialyseflüssigkeit oder des Blutes oder auch beide Kreisläufe dargestellt sein, wobei die darin enthaltenen Komponenten, wie z. B. Pumpen, Absperrvorrichtungen, Sensoren oder auch der Dialysator selbst, mittels charakteristischer Symbole darstellbar sind. Die Darstellung des gesamten Kreislaufs erleichtert die Orientierung des Benutzers bei der Auswahl eines oder mehrerer der Komponenten des Dialysekreislaufs. Die Wahrscheinlichkeit für die Verwechslung verschiedener Pumpen, Absperrvorrichtungen, oder z.B. Heizvorrichtungen wird auf diese Weise minimiert. Ebenso ist es möglich, daß nur ein Teil der Komponenten eines medizintechnischen Gerätes, insbesondere eines Dialysegerätes bzw. eines Dialysekreislaufes darstellbar ist. Dabei können zwei oder auch mehr beliebige Komponenten des Gerätes sowie deren funktioneller Zusammenhang dargestellt werden. Beispielsweise ist es denkbar, daß der Dialysator und die Blutpumpe sowie die beide Komponente verbindende Schlauchleitung mittels der zweiten Mittel anzeigbar sind. Die Auswahl der anzuzeigenden Kombinationen der Komponenten bzw. Ausschnitte aus der entsprechenden Gesamtanlage kann durch Grundeinstellungen des Gerätes festgelegt oder durch den Bediener je nach Bedarf gestaltbar sein. Durch die Darstellung der funktionellen Zusammenhänge der Komponenten wird die Bedienungsführung für diese Komponenten erleichtert.

In weiterer Ausgestaltung der Erfindung ist vorgesehen, daß die ersten und zweiten Mittel derart ausgeführt sind, daß gleichzeitig die Symbole darstellbar und die zugehörigen Behandlungsdaten anzeigbar und/oder veränderbar sind. Dadurch läßt sich die versehentliche Änderung eines Sollwertes oder die Aufnahme eines Istwertes verhindern, da stets gleichzeitig das Symbol der Komponente selbst sowie der zu verändernde Parameter angezeigt werden.

Die durch die ersten Mittel angezeigbaren und/oder veränderbaren Behandlungsdaten können die Bezeichnung sowie Soll- und/oder Istwerte und/oder Grenzwerte der Geräte- und/oder der Patientenparameter umfassen. Während im Rahmen eines Monitoring im wesentlichen die Istwerte der charakteristischen Daten der Komponenten abgefragt werden, läßt sich in einer weiteren Betriebsart der erfindungsgemäßen Vorrichtung eine gezielte Veränderung der Sollwerte durchführen. Dabei ist es möglich, nicht nur einzelne Sollwerte, sondern entsprechend auch eine zeitliche Abhängigkeit der Sollwerte und somit ein Sollwertprofil vorzugeben. Ebenso ist es möglich für die charakteristischen Daten Grenzwerte festzulegen, deren Einhaltung einen gefährlichen Betriebszustand sicher vermeidet. Das Erreichen der Grenzwerte kann dabei optisch oder akustisch signalisiert werden oder auch zu einem Abschalten des Gerätes bzw. zu einem Schließen von Absperrvorrichtungen führen. Die durch die ersten Mittel anzeigbaren und/oder veränderbaren Daten können darüber hinaus weitere Parameter und Werte umfassen, die z. B. den Verlauf der bisherigen Dialysebehandlung oder die abspeicherbaren Programmdaten vorangegangener Behandlungen verschiedener Patienten betreffen.

Besonders vorteilhaft ist es, wenn durch die Berührung der Symbole die Mittel zur Veränderung sowie die Mittel zur Anzeige der Behandlungsdaten gleichzeitig ansteuerbar sind. Auf diese Weise werden beispielsweise Kontrollflächen generiert, die gleichzeitig die aktuellen Istwerte der entsprechenden Parameter anzeigen, und ferner beispielsweise mit Pfeilen versehene Felderzur entsprechenden Änderung der Sollwerte umfassen. Die Veränderung von Daten erstreckt sich nicht nur auf eine Veränderung des Sollwertes, sondern kann beispielsweise auch eine komplette Ab- oder Einschaltung einer Komponente oder Funktionseinheit des Gerätes umfassen.

Ebenso ist es möglich, daß zunächst nach Berührung der charakteristischen Symbole der Komponenten des medizinischen Gerätes eine Anzeige der Behandlungsdaten erfolgt und daß die Mittel zur Veränderung der Daten durch entsprechende Berührung der anzeigbaren Behandlungsdaten ansteuerbar sind. Somit erfolgt nach einer einmaligen Berührung eines Symbols zunächst nurdie Anzeige der Daten und nurfür den Fall, daß diese verändert werden sollen ist eine erneute Berührung des Touchscreen-Monitors erforderlich.

Die Komponenten des medizintechnischen Gerätes, die mittels Symbolen darstellbar sind, können Blut- und Dialysatpumpen und/oder Mittel zur Dialysataufbereitung und/oder Sensoren umfassen. Dabei können die Sensoren sowohl als Temperaturals auch als Druckaufnehmer ausgeführt sein. Ebenso ist es möglich, daß das Dialysegerät selbst oder auch Absperreinrichtungen, wie Ventile oder Klemmen oder auch Heizvorrichtungen darstellbar sind.

Besonders vorteilhaft ist es, wenn durch die Berührung mehrerer Symbole gleichzeitig mehrere der zugehörigen Behandlungsdaten anzeigbar und/oder veränderbar sind. Auf diese Weise ist es möglich, beispielsweise einen Satz von Istwerten oder auch eine Auflistung der Sollwerte durch Berührung mehrerer oder aller Symbole zu erhalten. Ebenso kann auf diese Weise ein Profil der Istwertverläufe der bisher durchgeführten Behandlung oder auch vergangener Behandlungen erhalten werden.

In weiterer Ausgestaltung der Erfindung ist vorgesehen, daß die ersten Mittel derart ausgeführt sind, daß eine zeitliche Abhängigkeit der Behandlungsdaten vorgebbar ist. Auf diese Weise sind Sollwertprofile vorgebbar, wodurch beispielsweise im Rahmen einer Dialysebehandlung in der Regel den Behandlungserfolg gegenüberfestvorgegebenen Sollwerten verbessert werden kann. Dabei können die Sollwerte entsprechend mit den Zeitvorgaben in Form einer Tabelle oder einer ähnlichen Auflistung eingegeben werden. Auch ist es möglich, daß der Benutzer durch die Berührung eines entsprechenden Feldes der Touchscreen-Oberfläche einen gewünschten Verlauf des Sollwertes in Abhängigkeit der Zeit vorgibt. Eine Recheneinheit ermittelt in diesem Fall die gewünschten Sollwerte, die nach einer vorgebbaren Dialysezeit erreicht werden sollen.

Besonders vorteilhaft ist es, wenn die Touchscreen-Funktion nach Ablauf eines Zeitintervalls nach Ansteuerung oder Berührung der Touchscreen-Oberfläche abschaltbar ist. Dadurch wird erreicht, daß dann, wenn der letzte Bedienvorgang oder die letzte Ansteuerung der Touchscreen-Oberfläche eine definierte Zeit zurückliegt, die Touchscreen-Funktion ausgeschaltet wird, wodurch eine versehentliche Berührung keine nachteiligen Auswirkungen hat. Insbesondere kann es somit nicht mehr zu einerversehentlichen Veränderung der Anzeige oder gar zu einer Änderung der Sollwerte von Patienten- oder Geräteparametern kommen. Auch ist es möglich, daß gleichzeitig oder mit einer zeitlichen Verzögerung eine Anzeige generierbar ist, die eine Auswahl charakteristischer Daten umfaßt. Dadurch wird eine übersichtliche Anzeige ausschließlich relevanter Daten generiert, die es dem Bediener erleichtert, einen Behandlungsvorgang zu überwachen und Fehlfunktionen deutlich zu erkennen. Dabei ist die Auswahl der auf diese Weise anzeigbaren charakteristischen Daten durch den Bediener veränderbar oder durch das Gerät vorgebbar und richtet sich beispielsweise nach der Art der Behandlung. Eine besonders übersichtliche Darstellung wird erreicht, wenn die Auswahl charakteristischer Daten in einer vergrößerten Darstellung erfolgt,

Die vorliegende Erfindung betrifft ferner ein Verfahren zur Bedienung eines medizintechnischen Gerätes, insbesondere eines Dialysegerätes. Dabei werden die charakteristischen Behandlungsdaten durch einen Bildschirm mit Touchscreen-Oberfläche in einer ersten Bildschirmansicht angezeigt und/oder sind veränderbar, und in wenigstens einer zweiten Bildschirmansicht werden die für die Komponenten des Dialysegerätes charakteristischen Symbole dargestellt. Durch die Berührung dieser Symbole wird die erste Bildschirmansicht generiert. Das erfindungsgemäße Verfahren erfordert somit vom Benutzer ausschließlich das Berühren des gewünschten Symbols der Komponente, von dem entweder der Istwert oder ein sonstiger Parameter abgerufen werden soll oder ein Sollwert zu verändern ist. Nach dem Berühren des charakteristischen Symbols erfolgt in einer entsprechenden Bildschirmansicht beispielsweise die Anzeige der gewünschten Istwerte oder sonstiger Daten. Ebenso ist es möglich, daß nicht die Anzeige der Daten erfolgt, sondern eine Veränderung der Daten ermöglicht wird. Dies ist insbesondere dann anzustreben, wenn beispielsweise die Istwerte der Parameter stets zusammen mit den Symbolen der Komponenten des Gerätes angezeigt werden und eine erneute Darstellung dieser Werte nicht erforderlich ist. Nach der Anzeige oder Veränderung der Parameter kann die erste Bildschirmansicht geschlossen werden, und es erfolgt beispielsweise eine erneute Darstellung der Symbole der Komponenten des Gerätes.

In weiterer Ausgestaltung der Erfindung ist vorgesehen, daß durch gleichzeitiges oder aufeinanderfolgendes Berühren mehrerer Symbole eine Bildschirmanzeige generiert wird, die die gleichzeitige Anzeige oder Veränderung mehrerer Behandlungsdaten ermöglicht. Somit sind beispielsweise gleichzeitig die Istwerte sämtlicher Behandlungsparameter anzeigbar und somit leicht kontrollierbar.

Gemäß einer weiteren Ausgestaltung der vorliegenden Erfindung ist vorgesehen, daß die Berührung der Symbole die Anzeige der entsprechenden Behandlungsdaten generiert, und die Berührung der angezeigten Behandlungsdaten eine weitere Bildschirmanzeige erzeugt, die die Veränderung dieser Daten ermöglicht.

Besonders vorteilhaft ist es, wenn die Symbole der Komponenten des Gerätes stets gleichzeitig mit den zugehörigen Istwerten der Parameter dargestellt werden. Eine derartige Ausgestaltung des Verfahrens ermöglicht eine Übersicht über die aktuellen Daten, ohne daß dazu ein Eingreifen durch das behandelnde Personal notwendig ist. In diesem Fall ist es ausreichend, wenn die Berührung der entsprechenden Symbole nicht zu einer erneuten Anzeige des Istwertes führt, sondern beispielsweise die Veränderung des entsprechenden Sollwertes oder anderer Daten ermöglicht.

In einerweiteren Ausgestaltung der vorliegenden Erfindung ist vorgesehen, daß die Berührung der Symbole eine Bildschirmansicht generiert, durch die die zeitliche Abhängigkeit eines oder mehrerer der Behandlungsdaten dargestellt wird. Ist beispielsweise eine zeitlich variable lonenkonzentration einer Dialyselösung erforderlich, kann durch das Berühren eines entsprechenden Symbols auch die zeitliche Abhängigkeit dieses Sollwertes eingegeben werden.

Weitere Vorteile und Einzelheiten der vorliegenden Erfindung werden aus einem in der Zeichnung dargestellten Ausführungsbeispiel deutlich.

Es zeigen
- Fig. 1:: eine Bildschirmansicht mit einer symbolhaften Darstellung der Komponenten eines Dialysekreislaufs und
- Fig. 2:: eine Bildschirmansicht nach Berührung des Symbols der Blutpumpe in Fig. 1.

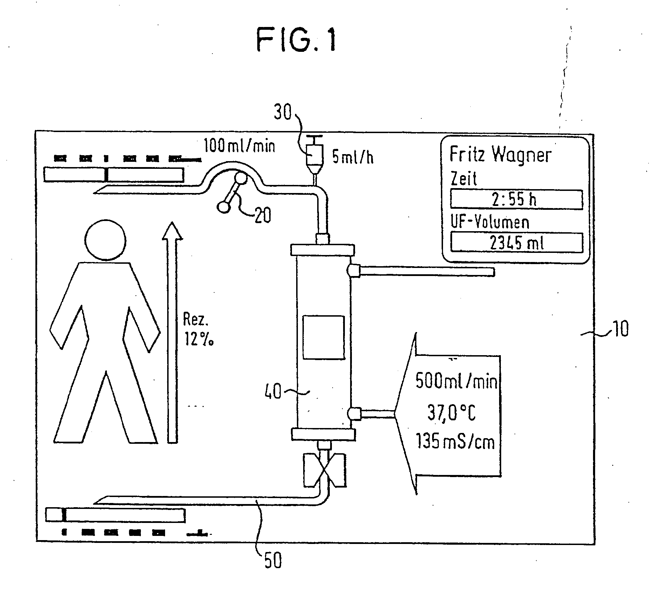

Fig. 1 zeigt die Touchscreen-Obertläche 10 der erfindungsgemäßen Vorrichtung mit den Symbolen für eine Blutpumpe 20, eine Spritzenpumpe 30 sowie den Dialysator 40. Diese Komponenten sind Bestandteile des extrakorporalen Kreislaufs 50, der im Doppelnadelmodus betrieben wird. Die neben dem Dialysatorsymbol 40 dargestellten Werte zeigen die Istwerte des Volumenstroms, der Temperatur und der Leitfähigkeit der zugeführten Dialyseflüssigkeit an.

Sollen beispielsweise die Parameter der Blutpumpe des Dialysekreislaufs 50 angezeigt oder verändert werden, genügt eine Berührung des Symbols 20 auf der Touchscreen-Oberfläche 10, um eine entsprechende Anzeige zu erhalten.

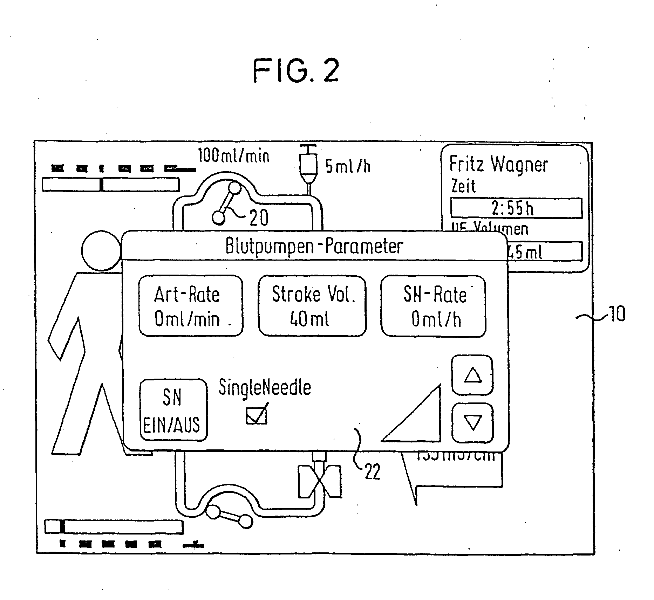

Fig. 2 zeigt die entsprechende Bildschirmansicht nach der Berührung des Symbols 20. Diese Bildschirmansichtzeigt in ihrem oberen Bereich das Symbol 20 der Blutpumpe und unmittelbar darunter ein Feld mit den zugehörigen Blutpumpen-Parametern. Hier dargestellt sind die Behandlungsdaten 22, wobei je nach gewünschter Betriebsart Istwerte oder Sollwerte angezeigt werden und mittels der Pfeiltasten eine entsprechende Veränderung der Sollwerte möglich ist. Zusätzlich kann gemäß dem vorliegenden Ausführungsbeispiel auch die Betriebsart der Blutpumpe zwischen Double-Needle- und Single-Needle-Betrieb geändert werden. Nach dem Einstellen der gewünschten Sollwerte oder Ablesen der erforderlichen Istwerte kann vorgesehen sein, daß das entsprechende Kontrollfeld entweder automatisiert nach einer vorgebbaren Zeitspanne oder durch die Berührung eines entsprechenden Feldes geschlossen wird, wodurch auf der Touchscreen-Oberfläche 10 erneut die in Fig. 1 dargestellte Ansicht erscheint.

Die erfindungsgemäße Vorrichtung und das erfindungsgemäße Verfahren zur Bedienung eines medizintechnischen Gerätes ermöglichen somit einen einfachen und zuverlässigen Betrieb des Gerätes, beispielsweise einer Dialysevorrichtung, und ermöglichen durch die eindeutige Symbolik der Gerätekomponenten und die Zuordnung entsprechender Kontrollfelder, die durch Berühren der Symbole erzeugbar sind, eine fehlerfreie Bedienung des Gerätes.

## Patentansprüche

1. Vorrichtung zur Bedienung medizintechnischer Geräte, insbesondere von Dialysegeräten, mit Bildschirm und Touchscreen-Oberfläche (10) sowie mit ersten Mitteln zur Anzeige und/oder Veränderung charakteristischer Behandlungsdaten (22), **dadurch gekennzeichnet, daß** zweite Mittel zur Darstellung von für die Komponenten des Gerätes charakteristischen Symbolen (20, 30, 40) sowie von deren funktionalen Zusammenhang vorgesehen sind, und die ersten Mittel durch Berührung der Symbole (20, 30, 40) auf der Touchscreen-Oberfläche (10) ansteuerbar sind.

2. Vorrichtung nach Anspruch 1, **dadurch gekennzeichnet, daß** wenigstens ein vollständiger Dialysekreislauf (50) mit den Symbolen (20, 30, 40) der darin enthaltenen Komponenten darstellbar ist.

3. Vorrichtung nach Anspruch 1 oder 2, **dadurch gekennzeichnet, daß** die ersten und zweiten Mittel derart ausgeführt sind, daß gleichzeitig die Symbole (20, 30, 40) darstellbar und die zugehörigen Behandlungsdaten (22) anzeigbar und/oder veränderbar sind.

4. Vorrichtung nach einem odermehreren der Ansprüche 1-3, **dadurch gekennzeichnet, daß** die durch die ersten Mittel anzeigbaren und/oderveränderbaren Behandlungsdaten (22), die Bezeichnung sowie Soll- und/oder Istwerte und/oder Grenzwerte der Geräte- und/oder der Patientenparameter umfassen.

5. Vorrichtung nach Anspruch 4, **dadurch gekennzeichnet, daß** durch Berührung der Symbole (20, 30, 40) die Mittel zurVeränderung und die Mittel zur Anzeige der Behandlungsdaten (22) gleichzeitig ansteuerbar sind.

6. Vorrichtung nach Anspruch 4, **dadurch gekennzeichnet, daß** die Mittel zur Veränderung der Behandlungsdaten (22) durch Berührung der anzeigbaren Behandlungsdaten (22) ansteuerbar sind.

7. Vorrichtung nach einem oder mehreren der Ansprüche 1-6, **dadurch gekennzeichnet, daß** die Komponenten des medizintechnischen Gerätes Blut- und Dialysatpumpen und/oder Mittel zur Dialysataufbereitung und/oder Sensoren umfassen.

8. Vorrichtung nach einem oder mehreren der Ansprüche 1-7, **dadurch gekennzeichnet, daß** durch die Berührung mehrerer Symbole (20, 30, 40) gleichzeitig mehrere der zugehörigen Behandlungsdaten (22) anzeigbar und/oder veränderbar sind.

9. Vorrichtung nach einem oder mehreren der Ansprüche 1-8, **dadurch gekennzeichnet, daß** die ersten Mittel derart ausgeführt sind, daß eine zeitliche Abhängigkeit der Behandlungsdaten (22) vorgebbar ist.

10. Vorrichtung nach einem oder mehreren derAnsprüche 1-9, **dadurch gekennzeichnet, daß** die Touchscreen-Funktion nach Ablauf eines Zeitintervalls nach Ansteuerung oder Berührung der Touchscreen-Oberfläche (10) abschaltbar ist.

11. Verfahren zur Bedienung medizintechnischer Geräte, insbesondere von Dialysegeräten, bei dem durch einen Bildschirm mit Touchscreen-Oberfläche (10) in einer ersten Bildschirmansicht charakteristische Behandlungsdaten (22) angezeigt werden und/oder verändert werden können, **dadurch gekennzeichnet, daß** in wenigstens in einer zweiten Bildschirmansicht für die Komponenten des Gerätes charakteristische Symbole (20, 30, 40) sowie deren funktionaler Zusammenhang dargestellt werden und die Berührung der Symbole (20, 30, 40) die erste Bildschirmansicht generiert.

12. Verfahren nach Anspruch 11, **dadurch gekennzeichnet, daß** durch gleichzeitiges oder aufeinanderfolgendes Berühren mehrerer Symbole (20, 30, 40) eine Bildschirmanzeige generiert wird, die die gleichzeitige Anzeige oder Veränderung mehrerer Behandlungsdaten (22) ermöglicht.

13. Verfahren nach Anspruch 11 oder 12, **dadurch gekennzeichnet, daß** die Berührung der Symbole (20, 30, 40) die Anzeige der Behandlungsdaten (22) generiert, und die Berührung der angezeigten Behandlungsdaten (22) eine weitere Bildschirmanzeige erzeugt, die die Veränderung der Behandlungsdaten (22) ermöglicht.

14. Verfahren nach einem oder mehreren der Ansprüche 11-13, **dadurch gekennzeichnet, daß** die Symbole (20, 30, 40) stets gleichzeitig mit den zugehörigen Istwerten der Parameter dargestellt werden.

15. Verfahren nach einem oder mehreren der Ansprüche 11-14, **dadurch gekennzeichnet, daß** die Berührung der Symbole (20, 30, 40) eine Bildschirmansicht generiert, durch die die zeitliche Abhängigkeit eines oder mehrerer der Behandlungsdaten (22) dargestellt wird.

## Claims

1. Apparatus for operating medical devices, in particular dialysis devices, with a screen and touchscreen surface (10) and with first means for displaying and/or changing characteristic treatment data (22), **characterized in that** second means are provided for presenting symbols (20, 30, 40) characteristic of the components of the device and their functional connection, and the first means can be triggered by touching the symbols (20, 30, 40) on the touchscreen surface (10).

2. Apparatus according to Claim 1, **characterized in that** at least one complete dialysis circuit (50) can be presented with the symbols (20, 30, 40) of the components which are included in it.

3. Apparatus according to Claim 1 or 2, **characterized in that** the first and second means are designed in such a way that the symbols (20, 30, 40) can be presented and the associated treatment data (22) displayed and/or changed simultaneously.

4. Apparatus according to one or more of Claims 1-3, **characterized in that** the treatment data (22) which can be displayed and/or changed by the first means include the identification and the desired and/or actual values and/or limit values of the device and/or patient parameters.

5. Apparatus according to Claim 4, **characterized in that** the means for changing and the means for displaying the treatment data (22) can be triggered simultaneously by touching the symbols (20, 30, 40).

6. Apparatus according to Claim 4, **characterized in that** the means for changing the treatment data (22) can be triggered by touching the displayable treatment data (22).

7. Apparatus according to one or more of Claims 1-6, **characterized in that** the components of the medical device include blood and dialysate pumps and/or means for dialysate preparation and/or sensors.

8. Apparatus according to one or more of Claims 1-7, **characterized in that**, by touching several symbols (20, 30, 40), several of the associated treatment data (22) can be displayed and/or changed simultaneously.

9. Apparatus according to one or more of Claims 1-8, **characterized in that** the first means are designed in such a way that a time dependence of the treatment data (22) can be predetermined.

10. Apparatus according to one or more of Claims 1-9, **characterized in that** the touchscreen function can be switched off on expiry of a time interval following the triggering or touching of the touchscreen surface (10).

11. Method for operating medical devices, in particular dialysis devices, in which method, using a screen with touchscreen surface (10), characteristic treatment data (22) can be displayed and/or changed in a first screen view, **characterized in that** symbols (20, 30, 40) characteristic of the components of the device and their functional connection are presented in at least one second screen view, and touching the symbols (20, 30, 40) generates the first screen view.

12. Method according to Claim 11, **characterized in that**, by touching several symbols (20, 30, 40) simultaneously or in succession, a screen display is generated which permits simultaneous display or changing of several treatment data (22).

13. Method according to Claim 11 or 12, **characterized in that** touching the symbols (20, 30, 40) generates the display of the treatment data (22), and touching the displayed treatment data (22) generates a further screen display which permits changing of the treatment data (22).

14. Method according to one or more of Claims 11-13, **characterized in that** the symbols (20, 30, 40) are at all times presented simultaneously with the associated actual values of the parameters.

15. Method according to one or more of Claims 11-14, **characterized in that** touching the symbols (20, 30, 40) generates a screen view with which the time dependence of one or more of the treatment data (22) is presented.

## Revendications

1. Dispositif de commande d'appareils médicaux, notamment d'appareils de dialyse, avec un écran et une surface à écran tactile (10) et avec des premiers moyens pour l'affichage et/ou la modification de données de traitement caractéristiques (22),
**caractérisé**
**en ce que** sont prévus des seconds moyens pour la représentation de symboles (20, 30, 40) caractéristiques des composants de l'appareil, ainsi que de leur rapport fonctionnel, et en ce que les premiers moyens peuvent être commandés par un contact des symboles (20, 30, 40) sur la surface à écran tactile (10).

2. Dispositif selon la revendication 1, **caractérisé en ce qu'**au moins une circulation de dialyse complète (50) avec les symboles (20, 30, 40) des composants contenus dans celle-ci peut être représentée.

3. Dispositif selon la revendication 1 ou 2, **caractérisé en ce que** les premiers et les seconds moyens sont réalisés de façon qu'en même temps les symboles (20, 30, 40) peuvent être représentés et les données de traitement associées (22) peuvent être affichées et/ou modifiées.

4. Dispositif selon l'une ou plusieurs des revendications 1 à 3, **caractérisé en ce que** les données de traitement (22) pouvant être affichées et/ou modifiées par les premiers moyens comprennent la désignation ainsi que des valeurs de consigne et/ou réelles et/ou des valeurs limites des paramètres se rapportant à l'appareil et/ou au patient.

5. Dispositif selon la revendication 4, **caractérisé en ce que** par le contact des symboles (20, 30, 40), les moyens pour la modification et les moyens pour l'affichage des données de traitement (22) peuvent être commandés simultanément.

6. Dispositif selon la revendication 4, **caractérisé en ce que** les moyens pour modifier les données de traitement (22) peuvent être commandés par un contact des données de traitement (22) pouvant être affichées.

7. Dispositif selon l'une ou plusieurs des revendications 1 à 6, **caractérisé en ce que** les composants de l'appareil médical comprennent des pompes et sang et de dialysat et/ou des moyens pour la préparation du dialysat et/ou des capteurs.

8. Dispositif selon l'une ou plusieurs des revendications 1 à 7, **caractérisé en ce que** par le contact de plusieurs symboles (20, 30, 40), simultanément plusieurs des données de traitement associées (22) peuvent être affichées et/ou modifiées.

9. Dispositif selon l'une ou plusieurs des revendications 1 à 8, **caractérisé en ce que** les premiers moyens sont réalisés de façon qu'une dépendance temporelle des données de traitement (22) peut être prédéfinie.

10. Dispositif selon l'une ou plusieurs des revendications 1 à 9, **caractérisé en ce que** la fonction de l'écran tactile peut être mise hors service après l'écoulement d'un intervalle de temps après la commande ou le contact de la surface à écran tactile (10).

11. Procédé de commande d'appareils médicaux, notamment d'appareils de dialyse, dans lequel, par un écran avec une surface à écran tactile (10), dans une première vue sur l'écran, des données de traitement caractéristiques (22) peuvent être affichées et/ou modifiées,
**caractérisé**
**en ce que** sont représentés sur au moins une deuxième vue sur l'écran des symboles (20, 30, 40) caractéristiques des composants de l'appareil, ainsi que leur rapport fonctionnel, et en ce que le contact des symboles (20, 30, 40) produit la première vue sur l'écran.

12. Procédé selon la revendication 11, **caractérisé en ce que** par un contact simultané ou successif de plusieurs symboles (20, 30, 40), une vue sur l'écran est produite qui permet l'affichage ou la modification simultané de plusieurs données de traitement (22).

13. Procédé selon la revendication 11 ou 12, **caractérisé en ce que** le contact des symboles (20, 30, 40) produit l'affichage des données de traitement (22), et que le contact des données de traitement affichées (22) produit un autre affichage sur l'écran qui permet la modification des données de traitement (22).

14. Procédé selon l'une ou plusieurs des revendications 11 à 13, **caractérisé en ce que** les symboles (20, 30, 40) sont représentés toujours simultanément avec les valeurs réelles associées des paramètres.

15. Procédé selon l'une ou plusieurs des revendications 11 à 14, **caractérisé en ce que** le contact des symboles (20, 30, 40) produit une vue sur l'écran par laquelle est représentée la dépendance temporelle d'une ou de plusieurs données de traitement (22).
